# NEW EUROPEAN PATENT SPECIFICATION

(11) **EP 0 925 789 B2**
(45) Date of publication and mention of the opposition decision: **17.06.2009**
(45) Mention of the grant of the patent: 21.07.2004
(21) Application number: 98309274.3
(22) Date of filing: 12.11.1998
(51) Int. Cl.: A61K 31/7052, A61P 27/02

(54) **USE OF AZITHROMYCIN IN THE TOPICAL TREATMENT OF OCULAR INFECTIONS**
VERWENDUNG VON AZITHROMYCIN ZUR TOPISCHEN BEHANDLUNG VON AUGENINFEKTIONEN
UTILISATION DE L'AZITHROMYCINE POUR LE TRAITEMENT TOPIQUE D'INFECTIONS OCULAIRES

(30) Priority: 02.12.1997 US 67250 P
(43) Date of publication of application: 30.06.1999
(73) Proprietor: Pfizer Products Inc., Groton, CT 06340 (US)
(72) Inventor: Ahmed, Imran, East Lyme, Connecticut 06333 (US)
(74) Representative: Kingsbury, Oliver William

(56) References cited:
- WO-A-95/09601
- WO-A-96/19489
- WO-A-96/20010
- WO-A1-96/39995
- US-A- 4 474 768
- US-A- 5 610 198
- JARURATANASIRIKUL, SUTEP ET AL: "Distribution of azithromycin into brain tissue, cerebrospinal fluid, and aqueous humor of the eye" ANTIMICROB. AGENTS CHEMOTHER., 1996, 40, 825-6, XP002098516
- BAILEY RL ET AL: "Randomised controlled trial of single-dose azithromycin in treatment of trachoma." LANCET, AUG 21 1993, 342 (8869) P453-6, XP002098517 ENGLAND
- THYLEFORS B.: "Azithromycin: A new opportunity for control of trachoma" WHO DRUG INFORMATION_(_WHO DRUG INF._), 10/3 (132-133), XP002098518 Switzerland
- DAWSON C.R.: 'Potential Use of Topical Azithromycin in Trachoma Control Programmes' FIRST MEETING OF THE WHO ALLIANCE FOR THE GLOBAL ELIMINATION OF TRACHOMA 1997, pages 1 - 4,23-24
- www.chemicalland21.com
- 'Römpp Chemie Lexikon, 9th Ed.', vol. 6, part T-Z 1992, GEORG THIEME VERLAG, STUTTGART page 4706
- 'Römpp Chemie Lexikon, 9th Ed.', vol. 1, part A-CI 1989, GEORG THIEME VERLAG, STUTTGART page 375

## Description

### Field of the invention

This invention relates to the use of azithromycin in the manufacture of a once daily topical medicament for treating eye infections.

### Background of the invention

Azithromycin is the U.S.A.N. (generic name) for 9a-aza-9a-methyl-9-deoxo-9a-homoerythromycin A, a broad spectrum antimicrobial compound derived from erythromycin A. Azithromycin is disclosed in U. S. patent no. 4,474,768 to Bright and U.S. patent no. 4,517,359 to Kobrehel et al. These patents disclose that azithromycin and certain derivatives thereof possess antibacterial properties and are accordingly useful as antibiotics.

Azithromycin is commonly administered orally, in a number of different dosage forms such as tablets, capsules, and suspensions, for the treatment of antibacterial infections. Until the present invention, however, azithromycin was not known to be effective when topically administered to the eye as described in Dawson, C.R.: first Meeting of the WHO Allience for the Global Elimination of Trachoma, Geneva, 30 June - 1 July 1997, pp 1-4 and 23-24. Azithromycin is known to be effective for the treatment of eye infections in humans when administered systemically, e.g., orally. However, it is also known that antibiotics which are effective when administered by a systemic route are not necessarily effective when applied topically, directly to the eye. For example, it has been reported that when tetracyclines are applied to the cornea, they do not penetrate the intact normal cornea even though they are able to diffuse into spinal fluid and into ocular fluids if the systemic dose is high enough (Douvas MG, et al, Arch Opthalmol. 46:57, 1951).

International Patent Application No. WO9620010 describes compositions comprising an anti-microbial agent, such as azithromycin, in combination with a second agent inhibiting methylation in a microorganism, and their use in the treatment of bacterial infections such as *chlamydia trachomatis*.

### Summary of the invention

This invention provides the use of azithromycin in the manufacture of a once daily topical medicament for treating an ocular infection.

The invention further provides a once daily pharmaceutical composition for topical application directly to an eye of an animal, including a human, said composition being suitable for the treatment of an ocular infection, comprising azithromycin as the sole active ingredient and a pharmaceutical vehicle suitable for topical application, wherein said azithromycin is at a concentration in said vehicle sufficient to remediate said ocular infection. In a preferred embodiment, the concentration of azithromycin in the vehicle is such that a single dose (approx. 5mg per eye) of said composition administered once daily for five days remediates the infection. It is noted that a "single dose" means the amount for a single eye. The capability of achieving once-a-day topical dosing with azithromycin was highly unexpected considering that most drugs are rapidly cleared from the precorneal area by tear drainage. Thus, commonly most topical regimens using known antibiotics such as gentamycin and erythromycin must be administered frequently with application rates of 4-6 times daily sometimes being required to produce effective drug levels in target ocular tissues. Topical formulations of azithromycin, by contrast, achieve relatively high and sustained levels in ocular tissues including cornea, conjuntiva, lids, and sclera. Because of the excellent penetration of azithromycin into ocular tissues, patient compliance is expected to be significantly enhanced by virtue of this invention.

The types of ocular infections treatable through the topical administration of azithromycin broadly include any eye infection caused by a bacterial species known to be amenable to systemic treatment with azithromycin. In particular, the invention is applicable to the treatment of trachoma, a chronic follicular conjunctivitis due to *Chlamydia trachomatis,* the world's leading cause of preventable blindness.

### Detailed description

The term "azithromycin" includes the pharmaceutically acceptable salts thereof, and anhydrous as well as hydrated forms. The azithromycin is preferably present as the dihydrate, disclosed, for example, in published European Patent Application 0 298 650 A2 and in co-pending U. S. application 07/994,040 filed December 21, 1992.

Compositions (sometimes referred to herein as "formulations") according to the invention comprise azithromycin and a pharmaceutically acceptable vehicle suitable for topical application to an eye. Azithromycin (calculated using the dihydrate) is typically present in the composition at a concentration of 0.1 to 2.5 weight % (w/w), usually 0.2 to 2.0 weight %, based on the weight of the composition. A preferred concentration is 0.5 weight %.

The compositions can include a preservative if desired, although preferred compositions do not contain a preservative. The compositions can also optionally contain surfactants, viscosity enhancers, buffers, sodium chloride, and water to form aqueous sterile ophthalmic solutions and suspensions. In order to prepare sterile ophthalmic ointment formulations, azithromycin is combined with an appropriate vehicle such as mineral oil, liquid lanolin, or white petrolatum. Sterile ophthalmic gel formulations containing azithromycin can be prepared by suspending azithromycin in a hydrophilic base prepared from a combination of, for example, a carboxyvinyl polymer sold under the designation Carbopol® (registered trademark of the B. F. Goodrich Company for a series of such polymers) according to published formulations for analogous ophthalmic preparations. Tonicity agents may also be incorporated in such gel formulations.

Azthromycin can be formulated as an ophthalmic solution in isotonic saline using glycerine as an isotonicity agent. A preservative can optionally be included as an excipient. Such ophthalmic solutions also include a pharmaceutically acceptable buffering agent, typically a combination of boric acid and sodium borate, sufficient to maintain the pH of the solution between 7 and 8.

A preferred composition is 0.5 % w/w azithromycin dihydrate suspended in an inert, non-allergenic, preservative-free vehicle consisting of white petrolatum (55% w/w), mineral oil (42.5% w/w), and lanolin (2% w/w).

The invention is further disclosed by means of the following examples. In the examples, reference to "water" means sterile water, suitable for use as water for injection.

### Example 1

A preferred embodiment was prepared by incorporating 5g of azithromycin dihydrate into 995 g of a sterile vehicle comprising 55% by weight of petrolatum, 43% by weight of light mineral oil, and 2% by weight of lanolin. The procedure involved first heating an excess amount of the ointment vehicle of the aforementioned composition to 70°C in a glass vessel to produce a melt. In the next step, 995g of the molten sterile ointment was transferred into a compounding vessel equipped with a mixer and 5g of the azithromycin hydrate was added to the melt under agitation at 70°C to form a suspension. The azithromycin containing ointment was rapidly cooled by placing the compounding vessel in an ice bath. The "0.5% azithromycin ointment" was then filled into 1cc unit dose plastic syringes for dose application. This ointment may be sterilized by gamma radiation using a cobalt-60 source.

### Example 2

A 0.5 weight percent azithromycin dihydrate opthalmic solution is prepared by dissolving 50g of azithromycin dihydrate (0.5 weight %), 67.0 g (0.67 weight percent) boric acid, 20.7 g (0.207 weight percent) sodium borate decahydrate, 100 g (1.0 weight percent) glycerin, 100 g of polyethylene glycol 300 (1.0 weight percent), and 0.40 g (0.004 weight percent) thimerosal (as a preservative) in about 8000 g of deionized distilled water. The pH is adjusted to 7.2 with HCI and NaOH. The final batch weight is brought to 10,000 g with the addition of the required amount of water. The final solution is filtered through a 0.2 micron Millipore filter and filtered into vials.

### Example 3

In a preferred embodiment, an approximate 0.5 weight percent azithromycin dihyrate opthalmic suspension is prepared as follows: 600 g of petrolatum is heated to 90°C for 2 hours in a jacketed 316 stainless steel vessel. The temperature is then decreased to 60°C. Light mineral oil, 350 g, is added to the petrolatum under mild agitation. The solution is passed through a sintered glass filter. Azithromycin dihyrate, 5g, is dispersed into the mineral oil/petrolatum solution under agitation to form a finely dispersed suspension. The suspension is cooled under slow agitation to form a semisolid suspension. The suspension is filled into plastic, polypropylene tubes and sterilized by gamma radiation using a cobalt-60 source.

### Example 4

A 0.5 weight percent azithromycin dihyrate opthalmic suspension is prepared as follows: 600 g of PEG 4000 is heated to 90°C for 2 hours in a jacketed 316 stainless steel vessel. The temperature is brought down to 60° C. PEG 400, 350 g, is added to the petrolatum under mild agitation. The solution is passed through a sintered glass filter. Azithromycin dihyrate, 50 g, is dispersed into the PEG 4000/PEG 400 solution under agitation to form a finely dispersed suspension. The suspension is cooled under slow agitation to form a semisolid suspension. The suspension is filled into plastic, polypropylene tubes and sterilized by gamma radiation using a cobalt-60 source.

### EXAMPLE 5

The following gel is prepared under strictly aseptic conditions:

| | % w/w |
|---|---|
| Azithromycin Dihydrate | 3.50 |
| Chlorbutol BP | 0.50 |
| Carbopol® 934P | 2.50 |
| NaOH (4% w/v solution) | 6.21 |
| Water | 87.29 |

Azithromycin dihydrate is dispersed in the sterile unneutralised Carbopol in water containing chlorbutol BP in solution. A sterile 4% w/v sodium hydroxide solution is then added with constant mixing to a final pH of 4-6.

### EXAMPLE 6

The following ingredients are formed into a mixture:

| | % w/w |
|---|---|
| Azithromycin Dihydrate | 3.50 |
| Chlorbutol BP | 0.50 |
| Citric acid monohydrate ** | 0.117 |
| Sodium citrate dihydrate ** | 0.112 |
| Sodium citrate 1 % solution ** | qs |
| Hydroxypropylmethylcellulose 2906 USP 4000 cps (sterile) | 3.80 |
| Water | to 100.00 |

| | |
|---|---|
| ** buffers | |

Citric acid, sodium citrate and chlorbutol BP are dissolved in 95% of the total water and the solution sterilized. Azithromycin powder is dispersed in the solution at ambient temperature using a high shear mixer. The hydroxypropylmethylcellulose, previously sterilized, is dispersed in the suspension and then allowed to hydrate over a period of about 15 minutes. The pH is adjusted to between 4-6 with a 1 % solution of sterilized sodium citrate. The gel is adjusted to final weight with water and mixed thoroughly.

### EXAMPLE 7

The following suspension, gelling in situ at body temperature, is prepared:

| | % w/w |
|---|---|
| Azithromycin Dihydrate | 3.50 |
| benzalkonium chloride BP | 0.02 |
| citric acid monohydrate | 0.117 |
| sodium citrate dihydrate | 0.112 |
| Pluronic® F127** | 19.00 |
| sodium citrate/citric acid solution | qs |
| water | to 100.00 |

| | |
|---|---|
| ** Pluronic® F127 is a polyoxyethylene-polyoxpropylene block copolymer of average molecular weight about 11,500 | |

Citric acid, sodium citrate and benzalkonium chloride are dissolved in 98% of the total water. The Pluronic® F127 is dispersed in this solution and left to hydrate ovemight. The preparation is then thoroughly mixed and the pH adjusted to 4-6 with sodium citrate or citric acid solution as appropriate. The solution is made to 96.5% of the total weight and sterile filtered into a sterile container. Azithromycin is dispersed aseptically in the filtered solution using a high shear mixer.

### EXAMPLE 8

The following gel is prepared under strictly aseptic conditions:

| | % w/w |
|---|---|
| Azithromycin Dihydrate | 3.50 |
| chlorbutol BP | 0.50 |
| ethylene maleic anhydride resin (EMA) type 91 (sterile) | 0.80 |
| dilute ammonium hydroxide solution (1.75% NH₃) | 4.40 |
| water | 90.80 |

The sterile EMA resin is dispersed in 50% of the total water, dilute ammonium hydroxide solution is stirred in and the mixture is heated at 95°C for 15 minutes. The resultant gel is allowed to cool to below 60°C.

The chlorbutol BP is dissolved in the remaining 50% of the water, at a temperature not exceeding 60°C, and sterile filtered into the gel which is mixed slowly.

Azithromycin is thoroughly dispersed in the gel.

## Claims

1. Use of azithromycin in the manufacture of a once daily topical medicament for treating an ocular infection.

2. A once daily pharmaceutical composition for topical application directly to an eye of an animal, including a human, said composition being suitable for the treatment of an ocular infection and comprising an effective amount of azithromycin as the sole active ingredient and a pharmaceutical vehicle suitable for topical application to the eye.

3. A composition as defined in claim 2, wherein azithromycin is present in an amount of 0.5 weight %.

4. A composition as defined in claim 2, wherein said composition is about 0.5 % w/w azithromycin dihydrate suspended in an inert, non-allergenic, preservative-free vehicle comprising white petrolatum (55% w/w), mineral oil (42.5% w/w), and lanolin (2% w/w).

## Patentansprüche

1. Verwendung von Azithromycin bei der Herstellung eines topischen Medikaments zur einmal täglichen Anwendung zur Behandlung einer Augeninfektion.

2. Pharmazeutische Zusammensetzung zur einmal täglichen Anwendung zur topischen Anwendung direkt am Auge eines Tiers, einschließlich eines Menschen, wobei die Zusammensetzung für die Behandlung einer Augeninfektion geeignet ist und eine wirksame Menge an Azithromycin als das alleinige aktive Ingrediens und ein pharmazeutisches Vehikel, das zur topischen Anwendung am Auge geeignet ist, umfasst.

3. Zusammensetzung, wie sie in Anspruch 2 definiert ist, in der Azithromycin in einer Menge von 0,5 Gew.-% vorliegt.

4. Zusammensetzung, wie sie in Anspruch 2 definiert ist, wobei die Zusammensetzung etwa 0,5% G/G Azithromycin-Dihydrat, suspendiert in einem inerten, nicht-allergenen, von Konservierungsmittel freien Vehikel, das weißes Petrolatum (55% G/G), Mineralöl (42,5% G/G) und Lanolin (2% G/G) umfasst, ist.

## Revendications

1. Utilisation d'azithromycine dans la fabrication d'un médicament topique à prise quotidienne unique pour traiter une infection oculaire.

2. Composition pharmaceutique à prise quotidienne unique destinée à une application topique directement sur l'oeil d'un animal, y compris un être humain, ladite composition convenant au traitement d'une infection oculaire et comprenant une quantité efficace d'azithromycine comme seul ingrédient actif et un véhicule pharmaceutique convenant à une application topique sur l'oeil.

3. Composition telle que définie dans la revendication 2, dans laquelle l'azithromycine est présente en une quantité de 0,5 % en poids.

4. Composition telle que définie dans la revendication 2, ladite composition consistant en environ 0,5 % (pds/pds) de dihydrate d'azithromycine en suspension dans un véhicule sans conservateur, non allergisant et inerte comprenant de la vaseline blanche [55 % (pds/pds)], de l'huile minérale [42,5 % (pds/pds)] et de la lanoline [2 % (pds/pds)].
